# EUROPEAN PATENT APPLICATION

(11) **EP 3 712 613 A1**
(43) Date of publication of application: **23.09.2020**
(21) Application number: 18880950.3
(22) Date of filing: 22.11.2018
(51) Int. Cl.: G01N 33/497, G01N 1/02, G01N 21/05, G01N 21/64, G01N 21/78, G01N 33/50, C12M 1/34, C12N 11/08

(54) **BIOGAS MEASUREMENT DEVICE**

(30) Priority: 22.11.2017 JP 2017225066
(71) Applicant: National University Corporation Tokyo Medical and Dental University, Bunkyo-ku Tokyo 113-8510 (JP)
(72) Inventor: MITSUBAYASHI, Kohji, Tokyo 113-8510 (JP); ARAKAWA, Takahiro, Tokyo 113-8510 (JP); TOMA, Koji, Tokyo 113-8510 (JP); SUZUKI, Takuma, Tokyo 113-8510 (JP); IITANI, Kenta, Tokyo 113-8510 (JP)
(74) Representative: EP&C
(86) International application number: PCT/JP2018/043291
(87) International publication number: WO 2019/103130

(57) **Abstract**

There is provided a biological gas measurement device that continuously collects biological gas, and is able to immediately and chronologically measure a target substance from the collected biological gas. A skin gas measurement device includes a biological gas collector 10 having an aperture portion 11 in a side thereof that faces a living body, and having a recessed portion 12 that is connected with the aperture portion 11 and serves as a space for collecting biological gas, a measurement device 100 that measures a target substance in the biological gas collected by the biological gas collector 10, an outflow path 40 through which collected biological gas is discharged from the recessed portion 12 to the measurement device 100, and a correction device 124 that corrects measurements of the target substance performed by the measurement device 100, and enables measurement results of the target substance from which effects of moisture present inside the outflow path 40 have been excluded to be output.

## Description

### Technical Field

The present invention relates to a biological gas (volatile organic components, VOCs) measurement device that measures a target substance contained in various types of biological gases such as skin gas and exhaled breath, human VOCs, gases released from mucous membranes such as conjunctiva, and evaporation gases from body fluids such as sweat and tears and the like that are secreted from inside the body.

### Background Art

Volatile compounds deriving from metabolism or from diseases are contained in biological gas, and it is thought that metabolic function evaluations and disease screenings can be achieved by performing non-invasive measurement of these volatile compounds. Skin gas, in particular, is suitable for being collected continuously, and is also suitable for gas concentration monitoring. However, compared to exhaled breath, the distribution coefficient of blood components in skin gas is extremely low, and the same is true for gases released from mucous membranes such as conjunctiva, and evaporation gases from body fluids such as sweat and tears and the like. Because of this, there is a demand for high-sensitivity measurement technology and selectivity. Although technologies disclosed in Japanese Patent Application Laid-Open (JP-A) Nos. H10-239309, 2002-195919, and 2010-148692 serve as examples of this type of technology, none of these technologies take into account harmful effects of moisture generated by sweating, so that problems in the measurement accuracy still exist.

### SUMMARY OF THE INVENTION

### Technical Problem

It is an object of each aspect of the present invention to provide a biological gas measurement device that enables a target substance to be measured with a high degree of accuracy from various types of biological gas such as skin gas and exhaled breath, gases released from mucous membranes such as conjunctiva, and evaporation gases from body fluids such as sweat and tears and the like that are secreted from inside the body, and, in particular, that enables biological gas to be collected continuously, and enables measurements of target substances from the collected biological gas to be performed immediately and chronologically, and that enables harmful effects from moisture which is present together with the biological gas to be excluded.

### Solution to the Problem

### (1) First Aspect

A biological gas measurement device according to a first aspect of the present invention comprises a biological gas collector having an aperture portion in a side thereof that faces a living body, and having a recessed portion that is connected with the aperture portion and serves as a space for collecting biological gas released either directly or indirectly from the living body, a measurement device that measures a target substance in the biological gas collected by the biological gas collector, an outflow path through which collected biological gas is discharged from the recessed portion to the measurement device, a moisture sensor that measures moisture present inside the recessed portion or the outflow path and a correction device that corrects measurements of the target substance performed by the measurement device, using measurements of moisture performed by the moisture sensor, and that enables measurement results of the target substance, from which effects of moisture present inside the outflow path have been excluded to be output.

The specific configuration of the 'biological gas collector' is not particularly restricted if the biological gas collector has the aforementioned aperture portion and the aforementioned recessed portion. Additionally, the biological gas collector may also be deformable such as being flexible or the like.

A type of the 'measurement device' is not particularly restricted if the measurement device is able to measure the target substance contained in the biological gas. For example, a biosensor that utilizes an enzymatic reaction of the target substance, or a semiconductor gas sensor that utilizes an antigen-antibody reaction of the target substance or the like may be used.

A type of the 'outflow path' that can be used is not particularly restricted if the outflow path allows the biological gas collected by the biological gas collector to be discharged to the measurement device. More specifically, the outflow path may be a tube that is connected with the recessed portion to the measurement device. Moreover, it is also possible for the outflow path to be formed integrally with the recessed portion in locations other than the aperture portion. In this case, a portion of the recessed portion doubles as the outflow path.

The 'moisture sensor' is a device that is able to detect moisture that is present inside the recessed portion and the flow path due to exhaled breath or sweating from a body surface. For example, a sweat sensor that is able to detect sweat secreted from a skin surface, or a humidity sensor that is able to detect water vapor contained in exhaled breath, or the like may be used as the moisture sensor.

The 'correction device' is a device that corrects the measurement of the target substance performed by the measurement device using a measurement of the moisture performed by the moisture sensor, and then enables a measurement result for the target substance, from which effects of moisture present inside the outflow path have been excluded, to be output. The correction device may be achieved, for example, via a computer system that performs processing on measurement data obtained by the measurement device and the moisture sensor using predetermined algorithms.

### (2) Second Aspect

A biological gas measurement device according to a second aspect of the present invention further comprises, in addition to the first aspect, an inflow port through which a gas flows into the recessed portion from outside, an outflow port through which a gas is discharged from the recessed portion to the outside, and an air supply device that supplies a carrier gas from the inflow port to the recessed portion via an inflow path, wherein the outflow path guides the biological gas discharged together with the carrier gas from the outflow port to the measurement device.

### (3) Third Aspect

A biological gas measurement device according to a third aspect of the present invention comprises a biological gas collector having an aperture portion in a side thereof that faces a living body, and having a recessed portion that is connected with the aperture portion and serves as a space for collecting biological gas released either directly or indirectly from the living body, a measurement device that measures a target substance in the biological gas collected by the biological gas collector, an outflow path through which collected biological gas is discharged from the recessed portion to the measurement device, and a correction device that corrects measurements of the target substance performed by the measurement device, and that enables measurement results of the target substance, from which effects of moisture present inside the outflow path have been excluded, to be output, wherein the measurement device comprises an enzyme membrane that is fitted onto an adjacent portion relative to the outflow path, at least a portion of the enzyme membrane being wetted by a solution containing a coenzyme, and the wetted portion being exposed to the biological gas and the moisture present inside the outflow path, and an irradiation unit that irradiates excitation light of a predetermined wavelength onto the solution, an enzyme that catalyzes a chemical reaction of the target substance which accompanies a chemical change in the coenzyme is fixed to the enzyme membrane, the coenzyme emits fluorescent light when excited by the excitation light, and the target substance is measured by fluorescence detection before and after the chemical change in the coenzyme, so that effects of moisture can be excluded by the correction device.

The 'enzyme' is selected from a variety of enzymes in accordance with the target substance contained in the biological gas. For example, if a primary alcohol dehydrogenase (ADH) is used as the enzyme, then the target substance is ethanol (coenzyme: oxidized nicotinamide adenine dinucleotide NAD⁺) or acetaldehyde (coenzyme: reduced nicotinamide adenine dinucleotide NADH). Moreover, if a secondary alcohol dehydrogenase (S-ADH) is used as the enzyme, then the target substance is acetone (coenzyme: NADH), or 2-propanol (coenzyme: NAD⁺) or the like. Furthermore, if aldehyde dehydrogenase (ALDH) is used as the enzyme, then the target substance is acetaldehyde or *trans*-2-nonenal (coenzyme: NAD⁺) or the like. If formaldehyde dehydrogenase (FALDH) is used, then the target substance is formaldehyde (coenzyme: NAD⁺).

### (4) Fourth Aspect

A biological gas measurement device according to a fourth aspect of the present invention further comprises, in addition to the third aspect, a clearance maintaining device that maintains a clearance between the aperture portion and the enzyme membrane at a fixed distance.

### (5) Fifth Aspect

A biological gas measurement device according to a fifth aspect of the present invention is characterized in that, in addition to the third or the fourth aspects, the measurement device further comprises a light-receiving unit that receives the fluorescent light.

### (6) Sixth Aspect

A biological gas measurement device according to a sixth aspect of the present invention is characterized in that, in addition to the fifth aspect, the irradiation unit is ring-shaped, and the light-receiving unit is provided on an inner side of the irradiation unit, and the irradiation unit and the light-receiving unit are positioned on a same side relative to the enzyme membrane.

### (7) Seventh Aspect

A biological gas measurement device according to a seventh aspect of the present invention further comprises, in addition to the fifth aspect or the sixth aspect, a detection unit that detects the target substance using fluorescent light received by the light-receiving unit.

### (8) Eighth Aspect

A biological gas measurement device according to an eighth aspect of the present invention is characterized in that, in addition to the seventh aspect, the detection unit detects the target substance by adding only a G channel and a B channel of an RGB color image received by the light-receiving unit.

### (9) Ninth Aspect

A biological gas measurement device according to a ninth aspect of the present invention further comprises, in addition to the seventh aspect or the eighth aspect, a visualization unit that visualizes spatial distribution information of a concentration of the target substance detected by the detection unit.

### (10) Tenth Aspect

A biological gas measurement device according to a tenth aspect of the present invention further comprises, in addition to any one of the first aspect to the ninth aspect, an adhering device that causes a periphery of the recessed portion to adhere to the living body.

The type of 'adhering device' is not particularly restricted if the adhering device is able to cause the periphery of the aperture portion to be tightly adhered to a living body (such as a wrist, an area around a mouth or an eye, or an ear or the like) so as to block off the recessed portion from the outside air, and, for example, an adhering device in which an adhesive material or an elastic body or the like is fitted around the periphery of the recessed portion can be used. Moreover, the periphery of the aperture portion can also be made to adhere directly to a living body by using a belt or a band that tightly fits the biological gas collector to a living body. Furthermore, the periphery of the aperture portion can be tightly adhered to a living body by forming the biological gas measurement device in the shape of a mask, goggles, earphones, headphones, or a hearing aid or the like.

### Advantageous Effects of the Invention

According to each aspect of the present invention, it is possible to measure target substances with a high degree of accuracy from various types of biological gas such as skin gas and exhaled breath, gases released from mucous membranes such as conjunctiva, and evaporation gases from body fluids such as sweat and tears and the like that are secreted from inside the body, and in particular, it is possible to continuously collect biological gas, and to take measurements of target substances from the collected biological gas immediately and chronologically, and it is also possible to exclude harmful effects that are caused by moisture which is present in the biological gas.

### BRIEF DESCRIPTION OF THE DRAWINGS

FIG 1 schematically shows a first exemplary embodiment of a biological gas measurement device of the present invention.
FIG 2 shows an enlargement of principal portions of the biological gas measurement device shown in FIG 1.
FIG 3A is a perspective view showing a biological gas collector that is used in the biological gas measurement device shown in FIG 1.
FIG 3B is a cross-sectional view showing a biological gas collector that is used in the biological gas measurement device shown in FIG 1.
FIG 3C is a bottom view showing a biological gas collector that is used in the biological gas measurement device shown in FIG 1.
FIG 4 schematically shows a cross-sectional view of the biological gas collector shown in FIG 3 in a mounted state.
FIG. 5 schematically shows a usage state of a second exemplary embodiment of the biological gas measurement device of the present invention.
FIG. 6 schematically shows a release mechanism of volatile components contained in blood.
FIG. 7 shows graphs contrasting relationships between an amount of sweat from skin (upper graph) and an amount of ethanol contained in skin gas (lower graph).
FIG. 8A shows graphs showing relationships between an amount of sweat from skin (upper graph) and an amount of ethanol contained in skin gas (lower graph) when a biological gas measurement device is fitted onto a palm of a hand.
FIG. 8B shows graphs showing relationships between an amount of sweat from skin (upper graph) and an amount of ethanol contained in skin gas (lower graph) when a biological gas measurement device is fitted onto a back of a hand.
FIG. 8C shows graphs showing relationships between an amount of sweat from skin (upper graph) and an amount of ethanol contained in skin gas (lower graph) when a biological gas measurement device is fitted onto a wrist of a hand.
FIG. 9 is a perspective view showing a third exemplary embodiment of the biological gas measurement device of the present invention.
FIG. 10A is a view schematically showing a state of use of a fourth exemplary embodiment of the biological gas measurement device of the present invention.
FIG. 10B is a cross-sectional view of a biological gas collector.
FIG. 11 schematically shows a usage state of a fifth exemplary embodiment of the biological gas measurement device of the present invention.
FIG. 12A is a front perspective view showing a sixth exemplary embodiment of the biological gas measurement device of the present invention.
FIG. 12B is a back perspective view showing the sixth exemplary embodiment of the biological gas measurement device of the present invention.
FIG. 12C is a side schematic view showing the sixth exemplary embodiment of the biological gas measurement device of the present invention.
FIG. 13A is a front view showing an excitation light irradiation unit and a light-receiving unit for fluorescent light in a measurement device that is used in the biological gas measurement device shown in FIG. 12A through FIG. 12C.
FIG. 13B is a (right) side view showing an excitation light irradiation unit and a light-receiving unit for fluorescent light in the measurement device that is used in the biological gas measurement device shown in FIG. 12A through FIG. 12C.
FIG. 14A is a front view showing a clearance maintaining device that is used in the biological gas measurement device shown in FIG. 12A to FIG. 12C.
FIG. 14B is a side view showing the clearance maintaining device that is used in the biological gas measurement device shown in FIG. 12A to FIG. 12C.
FIG. 14C is a back view showing the clearance maintaining device that is used in the biological gas measurement device shown in FIG. 12A to FIG. 12C.
FIG. 14D is a perspective view showing a usage example of the clearance maintaining device that is used in the biological gas measurement device shown in FIG. 12A to FIG. 12C.
FIG. 14E is a side view showing a usage example of the clearance maintaining device that is used in the biological gas measurement device shown in FIG. 12A to FIG. 12C.
FIG. 14F is a front view showing another example of a clearance maintaining device.
FIG. 14G is a side view showing another example of a clearance maintaining device.
FIG. 14H is a back view showing another example of a clearance maintaining device.
FIG. 14I is a perspective view showing a usage example of another example of a clearance maintaining device.
FIG. 15 schematically shows a method of creating a high-sensitivity visual form of an amount of ethanol contained in skin gas using the biological gas measurement device shown in FIG. 12A to FIG. 12C.
FIG. 16 schematically shows an amount of ethanol contained in skin gas which is visualized using the biological gas measurement device shown in FIG. 12A to FIG. 12C.
FIG. 17 is a perspective view showing a seventh exemplary embodiment of the biological gas measurement device of the present invention.
FIG. 18 is a perspective view showing an eighth exemplary embodiment of the biological gas measurement device of the present invention.

### DESCRIPTION OF THE EMBODIMENTS

### (1) First Exemplary Embodiment

### (1-1) Overview

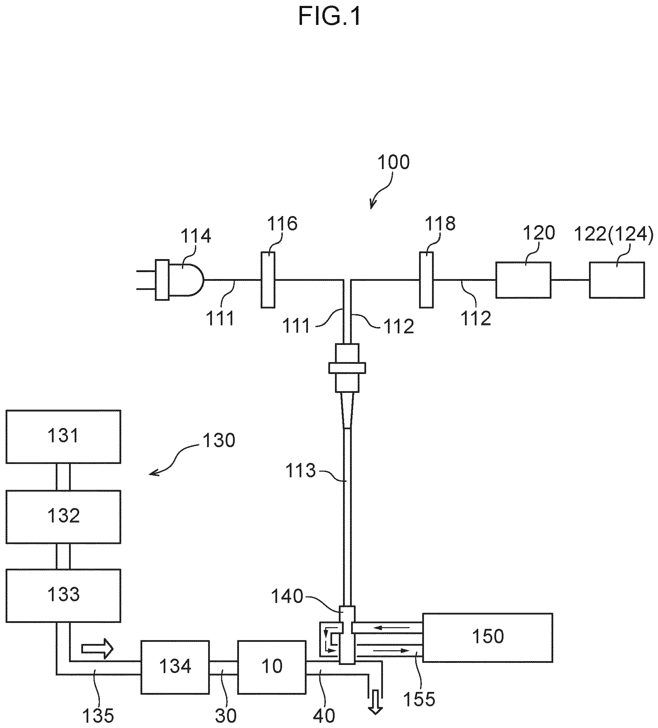

FIG. 1 is a view schematically showing a first exemplary embodiment of the biological gas measurement device of the present invention.

A biological gas measurement device of the present exemplary embodiment is provided with a biological gas collector 10 that collects skin gas as one form of biological gas, a measurement device 100 that measures a target substance contained in the skin gas collected by the biological gas collector 10, and an outflow path 40 that enables collected skin gas to be discharged from the biological gas collector 10 to the measurement device 100.

In the present exemplary embodiment, the target substance contained in the skin gas is taken as ethanol. The measurement of ethanol as the target substance utilizes a reaction in which a coenzyme NADH is produced from a coenzyme NAD⁺ in accompaniment to a reaction in which ethanol as a substrate is converted into acetaldehyde by alcohol dehydrogenase. More specifically, a phenomenon is utilized in which the coenzyme NADH, which is produced in conjunction with the above-described reaction, absorbs ultraviolet light having a wavelength of 340 nm, which serve as excitation light, and are consequently excited thereby and, as a result, emit fluorescent light having a wavelength of 491 nm. This is described below in greater detail.

### (1-2) Measurement Device 100

The measurement device 100 has an optical fiber probe 113 in which are combined an irradiation unit 111 that is formed by a light-irradiating optical fiber, and a light-receiving unit 112 that is formed by a light-receiving optical fiber. Commercially available instruments can be used for the optical fiber probe 113 and, for example, a combination of a 2-in-1 Optical Fiber Assembly (BIF600-UV/VIS) sold by Ocean Optics Inc. (US) and an F100-9009 (manufactured by Ocean Optics Inc.) can be used.

An ultraviolet light-emitting diode 114 that is used to irradiate ultraviolet light, which serve as excitation light having a predetermined wavelength in accordance with the excitation phenomenon being used, is connected to the irradiation unit 111. In addition, a bandpass filter 116 is connected between the ultraviolet light-emitting diode 114 and the optical fiber probe 113. In the present exemplary embodiment, because the ultraviolet light-emitting diode 114 is used as a light source, the device can be simplified and manufactured at a lower cost compared to when a mercury lamp is used as the light source. In addition, the device can also be used as a portable device.

As described above, because the present exemplary embodiment utilizes the property of NADH of absorbing 340 nm ultraviolet light, an ultraviolet light-emitting diode 114 that emits ultraviolet light having a wavelength of 300 to 370 nm, and preferably in the vicinity of 340 nm, is used as the ultraviolet light-emitting diode 114. Accordingly, as shown in FIG. 1, it is preferable that the bandpass filter 116 be connected between the ultraviolet light-emitting diode 114 and the optical fiber probe 113. The bandpass filter 116 is a filter that only allows light having a specific wavelength out of the light from the light source to be transmitted and, in the present exemplary embodiment, for example, a bandpass filter that transmits 330 to 350 nm ultraviolet light out of the incident ultraviolet light having a wavelength of 300 to 370 nm from the ultraviolet light-emitting diode 114 is used. A commercially available bandpass filter can be used without any particular restrictions as the bandpass filter 116.

In the biological gas measurement device shown in FIG. 1, the light-receiving unit 112 receives fluorescent light generated as a result of the excitation generated by the excitation light emitted from the ultraviolet light-emitting diode 114. Moreover, a detection unit 120 that detects the received fluorescent light is connected to the light-receiving unit 112. More specifically, a spectrophotometer that includes a photomultiplier tube, a photodiode detector and the like is used for the detection unit 120. The fluorescent light generated by this excitation has a specific wavelength that differs from the aforementioned predetermined wavelength in accordance with the above-described excitation phenomenon. In the case of NADH, this specific wavelength is 450 to 510 nm, and more specifically, is in the vicinity of 491 nm. Accordingly, as shown in FIG. 1, it is preferable that a long wavelength transmission filter 118 that only transmits fluorescent light having a greater wavelength than the specific wavelength be provided. In FIG. 1, a long wavelength transmission filter 118 that transmits fluorescent light having a wavelength of 400 nm or more is used. A commercially available long wavelength transmission filter can be used without any particular restrictions as the long wavelength transmission filter 118. Moreover, in the biological gas measurement device shown in FIG. 1, although the long wavelength transmission filter 118 is used, instead of the long wavelength transmission filter, it is also possible for a bandpass filter to be used. In this case, for example, a bandpass filter that only allows fluorescent light having a wavelength of 450 to 510 nm to be transmitted is used.

It is also possible for a computer system 122 to be further connected to the biological gas measurement device shown in FIG. 1. This computer system 122 analyzes data detected by the detection unit 120, and performs real-time displays of changes in concentration of the ethanol which is serving as the target substance of the present exemplary embodiment, and also performs data correction. Moreover, by connecting this type of computer system 122, and enabling it to also function as a correction device 124, it becomes a simple task to correct measurements of the ethanol performed by the measurement device 100 and to output measurement results for the ethanol from which effects from moisture which is present inside the outflow path 40 have been excluded, and to also perform data analysis. Note that the method used to perform such corrections may involve, for example, smoothing any abrupt peaks in the data that are due to effects of moisture, or by making corrections while considering any correlation with data that has been measured in advance relating to chronological changes in ethanol which is contained in blood or in exhaled breath.

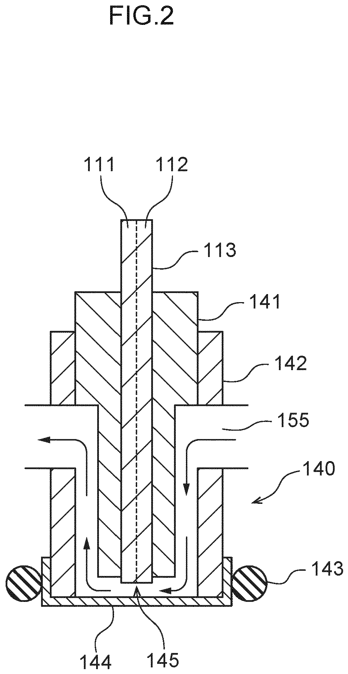

Next, the optical fiber probe 113 used in the biological gas measurement device shown in FIG. 1 will be described. As shown in FIG. 1, a container body 140, which serves as a gas-liquid diaphragm flow cell provided with an enzyme membrane 144 (see FIG. 2) to which an enzyme has been fixed, is attached to a distal end of the optical fiber probe 113. As shown in FIG. 2, this container body 140 may also include as part of its structure a silicon tube 141 and a PMMA (polymethyl methacrylate) pipe 142. Moreover, as shown in FIG. 2, the enzyme membrane 144 is fitted onto a distal end, which is one end side of the container body 140. This enzyme membrane 144 may also be fixed to the PMMA pipe 142 by means of an O-ring 143. A buffer solution which serves as a solution is contained in a reaction portion 145, which is an internal space inside the container body 140. This buffer solution contains NAD⁺ as the aforementioned coenzyme, and is supplied from a buffer solution reservoir 150 (see FIG. 1) to the reaction portion 145 via a buffer solution flow path 155. From there, the buffer solution is recirculated back to the buffer solution reservoir 150 via the buffer solution flow path 155. On the other hand, as shown in FIG. 2, the optical fiber probe 113 which is formed by combining the irradiation unit 111 with the light-receiving unit 112 is inserted through a rear end side, which is another end side of the container body 140. A distal end of the optical fiber probe 113 is inserted as far as the reaction portion 145 so as to come into contact with the buffer solution, and not only irradiates the excitation light inside the buffer solution, but also receives the fluorescent light. As shown in FIG. 1, the distal end of this container body 140, which is the one end side thereof, is fitted so as to be adjacent to the outflow path 40 through which a carrier gas (described below) that contains the skin gas collected by the biological gas collector 10 is discharged, and the enzyme membrane 144 which is fitted onto the distal end of the container body 140 which is the portion thereof that is adjacent to the outflow path 40, is thereby exposed to this carrier gas. In other words, by fitting the distal end of the container body 140 in such a way that this distal end is inserted into the outflow path 40, the carrier gas can be partitioned from the buffer solution via the enzyme membrane 144.

Next, the enzyme membrane 144 will be described. The enzyme membrane 144 is a membrane in which an enzyme is immobilized onto a carrier which is formed by a membrane material. The enzyme catalyzes a chemical reaction of the target substance which accompanies a chemical change in the coenzyme that is contained in the buffer solution which is serving as the solution. A material that is conventionally used to immobilize an enzyme can be used without any particular restrictions as the carrier. Examples of this type of material include resins such as polytetrafluoroethylene, polydimethylsiloxane, polypropylene, polyethylene, polymethyl methacrylate, and polystyrene, and fibers such as cotton and the like. Although the thickness of this carrier is not particularly restricted, it is preferably between 100 nm and 200 µm, and more preferably between 10 µm and 100 µm. The method used to produce a membrane on which an enzyme capable of being used in the biological gas measurement device of the present exemplary embodiment is immobilized is described, for example in JP-A No. 2009-168671. In addition, materials capable of being used as a carrier are described in JP-A No. 2016-220573, and the contents of these specifications are incorporated by reference into the present specification. However, the enzyme membrane 144 used in the present exemplary embodiment must have at least a portion thereof wetted by a solution containing the coenzyme.

The biological gas measurement device of the present exemplary embodiment quantifies the concentration of NADH using fluorescent light generated when the coenzyme (specifically, NADH) that has undergone the chemical change is excited by excitation light emitted from the ultraviolet light-emitting diode 114, and, using this concentration as an index, detects ethanol which is the substrate of the alcohol dehydrogenase that uses NADH as the coenzyme. Accordingly, it is necessary for the substrate contained in skin gas to react in the presence of a coenzyme with the enzyme contained in the enzyme membrane 144. In the biological gas measurement device shown in FIG. 1, by employing a structure in which the enzyme membrane 144 is interposed between the carrier gas which contains the skin gas and is flowing through the outflow path 40 and the buffer solution which contains NAD⁺ and is flowing through the reaction portion 145, the enzyme and the substrate are able to react in the presence of NAD⁺ which is a coenzyme. Accordingly, it is preferable that the carrier forming the enzyme membrane 144 is porous. Although there is no particular restriction on the size of the holes in the carrier provided that they allow an enzyme reaction to occur, the holes normally have a diameter of approximately 0.1 ∼ 1 µm, and from the standpoint of the enzyme reaction efficiency , it is preferable that the hole diameter be approximately 0.2 µm. It is also preferable that the porosity ratio of the carrier be between 60 and 90%. Note that a mesh-shaped material can be used as the carrier.

### (1-3) Air Supply Device 130

In the present exemplary embodiment, as shown in FIG. 1, an air supply device 130 that supplies a fixed quantity of carrier gas continuously to the biological gas collector 10 may include a compressor 131, an activated carbon filter 132, a gas generator 133, and a mass flow controller 134. The type of carrier gas is not particularly restricted provided that it is a gas that is inactive with respect to skin gas, and air may be used for the carrier gas. The carrier gas flows through a gas flow path 135 and flows from an inflow path 30 into the biological gas collector 10. There, it is mixed with skin gas, and comes into contact with the enzyme membrane 144 at the distal end of the container body 140 in the outflow path 40. At this time, when the ethanol which is serving as an enzyme substrate contained in the skin gas is oxidized by the alcohol dehydrogenase, the NAD⁺ contained in the buffer solution is reduced and, as described above, this causes fluorescent light to be emitted. As a result, the amount of ethanol contained in the skin gas can be measured. By using this type of air supply device 130, skin gas can be brought into continuous contact both efficiently and stably with the enzyme membrane 144.

### (1-4) Biological gas Collector 10

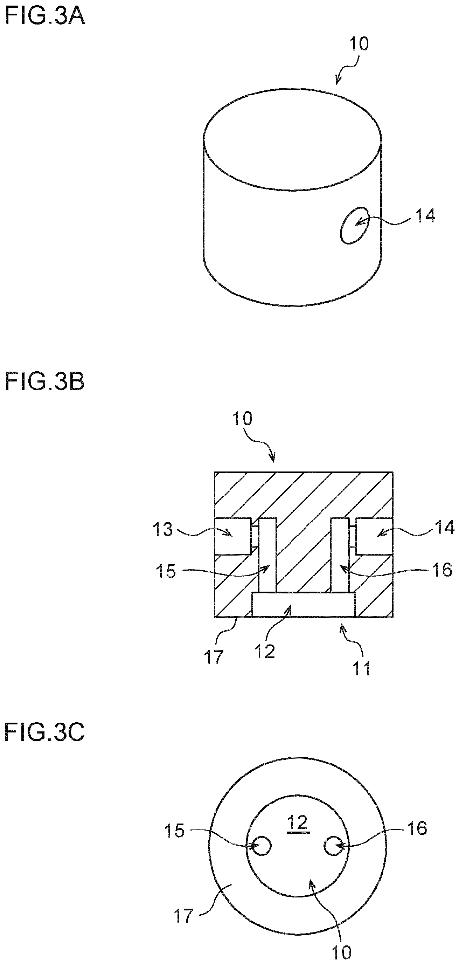

As shown in FIG. 3A, the biological gas collector 10 has a substantially circular-column shaped exterior appearance. As shown in FIG. 3B, an inflow port 13 and an outflow port 14 are formed facing each other in a side surface thereof. Moreover, recessed portion 12 (see FIG. 3B and FIG. 3C) that has an aperture portion 11 (see FIG. 3B), and continues on from this aperture portion 11 is also formed in a body contacting surface 17 which is a bottom surface of the biological gas collector 10 that is placed in contact with a living body. The recessed portion 12 is a space where skin gas emitted from the skin with which the body contacting surface 17 is in contact is collected. Moreover, the inflow port 13 and the outflow port 14 extend in parallel with the bottom surface. Furthermore, by providing an inflow communicating path 15 (i.e., a part of the inflow path 30 shown in FIG. 1) that enables the inflow port 13 to communicate with the recessed portion 12 in an axial direction, a gas (i.e., carrier gas) is able to flow from the outside into the recessed portion 12 through the inflow port 13. At the same time, by providing an outflow connecting path 16 (i.e., a part of the outflow path 40 shown in FIG 1) that enables the outflow port 14 to communicate with the recessed portion 12 in the axial direction, a gas (i.e., carrier gas and skin gas) is able to flow from the recessed portion 12 to the outside through the outflow port 14.

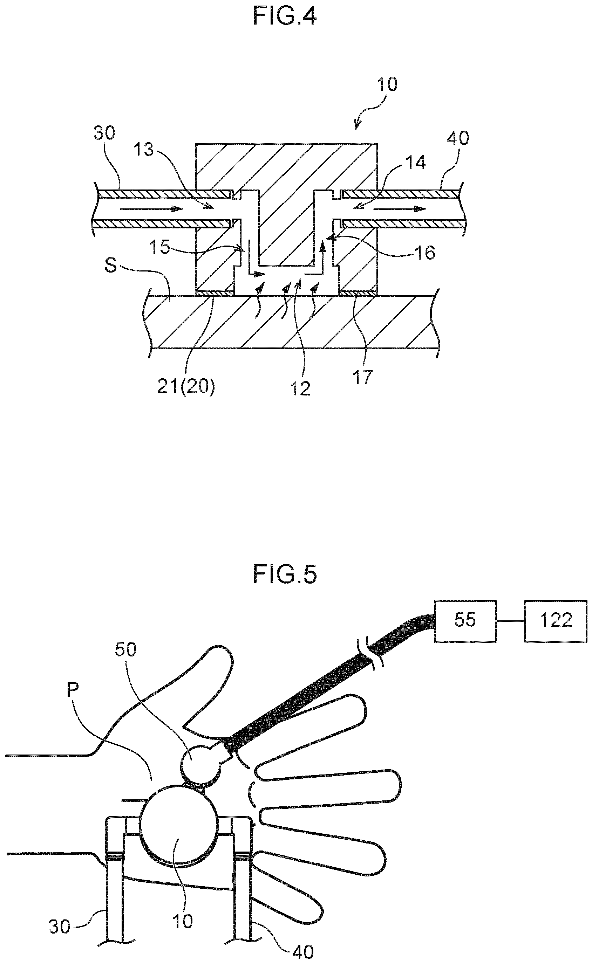

As shown in FIG. 4, double-sided tape 21 which serves as an adhering device 20 that enables the aperture portion 11 (see FIG. 3B) to be tightly adhered to the skin S with which it is in contact is adhered to the body contacting surface 17 of the biological gas collector 10. Skin gas is prevented by this adhering device 20 from leaking from the aperture portion 11 to the outside. Note that, in order to ensure tight adhesion and enable the adhering device 20 to approximately follow the contours of the living body, it is preferable that a tape having as a substrate a closed cell foam body through which gas is unable to permeate be used as the double-sided tape 21.

The inflow path 30 is connected to the inflow port 13, and carrier gas supplied from the air supply device 130 flows into the recessed portion 12 through this inflow path 30. Skin gas emitted from the skin S mixes with the carrier gas in the recessed portion 12. On the other hand, the outflow path 40 is connected to the outflow port 14, and a mixture gas formed by the carrier gas and the skin gas is guided by the inflow pressure of the carrier gas along the outflow path 40 to the measurement device 100.

### (1-5) Measurement by the Measurement Device 100

As described above, the mixture gas of skin gas and carrier gas is in contact with the distal end of the container body 140 in the outflow path 40. The enzyme membrane 144 to which alcohol dehydrogenase has been fixed as an enzyme is fitted onto the distal end of the container body 140, and a buffer solution containing NAD⁺, which is a coenzyme of alcohol dehydrogenase, is circulated in the reaction portion 145 on either side of this enzyme membrane 144. The enzyme membrane 144 is wetted by the buffer solution, and if ethanol which is serving as a substrate is contained in the skin gas that is in contact with the enzyme membrane 144, then this ethanol is converted into acetaldehyde by the alcohol dehydrogenase. At this time, the coenzyme NAD⁺ is converted into NADH. Meanwhile, excitation light having a wavelength of 340 nm is continuously irradiated from the irradiation unit 111 inside the buffer solution in the reaction portion 145, so that the NADH converted from the NAD⁺ absorbs this excitation light and is excited thereby and, as a result, emits fluorescent light having a wavelength of 491 nm. The light-receiving unit 112 then receives this fluorescent light and this is then digitized as, for example, a fluorescence intensity by the detection unit 120.

When the above-described measurement is being performed, it is desirable that a gas having a known ethanol concentration be measured in advance, and that a correlation between the ethanol concentration and the fluorescence intensity be obtained as a calibration curve.

In this way, in the first exemplary embodiment of the biological gas measurement device of the present invention, skin gas is placed in contact with the enzyme membrane 144 which has been wetted by a buffer solution, and the ethanol is measured. Because of this, even if moisture from sweat and the like is contained in the mixture gas of skin gas and carrier gas, the enzyme membrane 144 which has been wetted from the start remains unaffected by such moisture. Accordingly, it is possible to measure accurately and stably the quantity of ethanol in a living body using skin gas. Moreover, the measurement device 100 of the first exemplary embodiment has superior advantages in that, unlike measurements performed using such other devices such as semiconductor gas sensors or the like, the measurement device 100 is not prevented from making measurements because of moisture, and does not obtain measurement results that are not accurate. Note that it is also possible to detect other substrates by using other dehydrogenases which use this coenzyme NAD⁺. Moreover, if the fluorescence property is already known, then it is also possible to detect other substrates via an enzyme reaction which uses another coenzyme instead of the above-described NAD⁺. In other words, the present invention is not limited to ethanol, and it is possible to use an enzyme and coenzyme in accordance with the substrate that is to be detected, and which is the target substance contained in the biological gas. For example, if the target substance is acetone, then S-ADH may be used as the enzyme, and NADH as the coenzyme. In this case, NADH is combined with S-ADH, and when the acetone reacts with the S-ADH, the NADH is oxidized into NAD⁺. Although NADH is excited by ultraviolet light having a wavelength of 340 nm so as to emit fluorescent light having a wavelength of 491 nm, the intensity of the fluorescent light is decreased in conjunction with this oxidation into NAD⁺. Accordingly, the acetone concentration can be measured using the difference in the fluorescent light intensity.

### (2) Second Exemplary Embodiment

FIG. 5 is a view schematically showing a second exemplary embodiment of the biological gas measurement device of the present invention. In the present exemplary embodiment, the same biological gas collector 10 as that of the above-described first exemplary embodiment is used and, in the same way as in the first exemplary embodiment, the inflow path 30 and the outflow path 40 are connected thereto. Note that the air supply device 130 that is connected to the inflow path 30 and the measurement device 100 that is connected to the outflow path 40 are the same as in the above-described first exemplary embodiment, and are accordingly not shown in the figure.

The present exemplary embodiment differs from the above-described first exemplary embodiment in that there is further provided a moisture sensor 50 that measures moisture present within the recessed portion 12 (see FIG. 3B) in the biological gas collector 10. This moisture sensor 50 confirms moisture that is present because of sweat emitted from skin and, more specifically, is a sweat sensor. Because of this, a sweat meter 55 that measures the amount of sweat is connected to the moisture sensor 50. In addition, the computer system 122 is also connected so as to serve as the correction device 124 that is used to analyze the sweat amount data, and correct the original data for the amount of ethanol obtained by the measurement device 100 by taking this sweat amount data into consideration. By connecting the computer system 122 in this way, the measurement of the target substance can be corrected based on the measurement of the moisture contained in the recessed portion 12 by the moisture sensor 50. Moreover, because measurement results from which effects of moisture present within the outflow path 40 through which the skin gas is discharged from the recessed portion 12 to the measurement device 100 have been excluded are output, analyzing the data becomes easier. Accordingly, compared to the first exemplary embodiment in which corrections are made without using the moisture sensor 50, the second exemplary embodiment enables more accurate measurements to be performed. Note that the computer system 122 may be the same one that is connected to the measurement device 100, or may be a different computer system. In addition, it is also possible for the moisture sensor 50 to be connected to the outflow path 40 so as to be able to directly measure the moisture content within the outflow path 40.

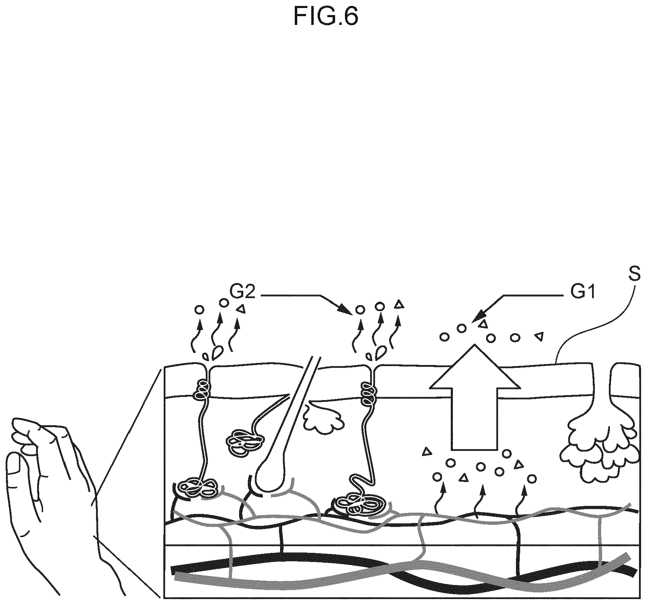

FIG. 6 schematically shows a release mechanism for volatile components in blood. Skin permeable gas G1 which is released by permeating the dermis and the epidermis, and sweat gas G2 which is released from sweat glands and volatized are contained in skin gas which includes volatile components in blood. Because of this, if the biological gas collector 10 (see FIG. 5) is adhered to the skin S, a mixture gas of the skin permeable gas G1 and the sweat gas G2 is collected, and blood derived components distributed in both are mixed together. Therefore, next, the measurement of the ethanol contained in skin gas (i.e., the skin permeable gas G1 and the sweat gas G2) released from a palm P of a hand using the second exemplary embodiment shown in FIG, 5 will be described.

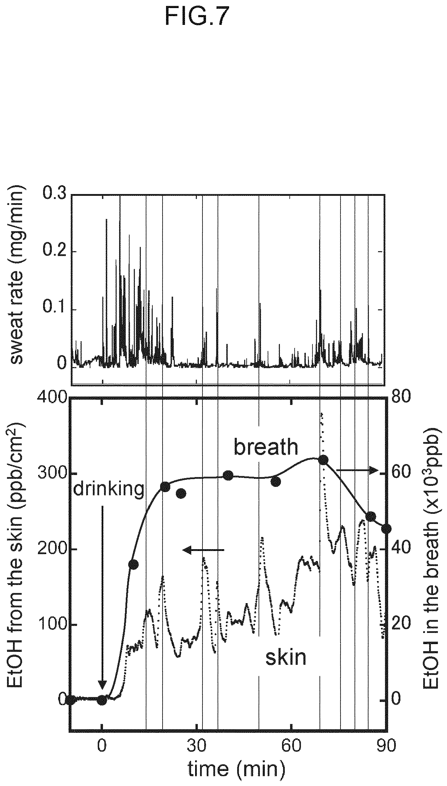

In FIG. 7, the amount of sweat from skin and the amount of ethanol contained in the skin gas (i.e., original data) when the biological gas collector 10 and the moisture sensor 50 were attached to a palm P, as shown in FIG. 5, are visualized as contrasting graphs. The biological gas collector 10 and the moisture sensor 50 were attached to the palm P of a seated test subject. In this state, the test subject ingested 0.4g/kg of body weight of ethanol by drinking alcohol. The lower graph shows the resulting amount of ethanol in the skin gas (i.e., the left-side vertical axis) and in the exhaled breath (i.e., the right-side vertical axis) in a chronological sequence. Note that the upper graph shows the amount of sweat from the skin which was measured at the same time. Here, the area of the aperture portion 11 of the biological gas collector 10 (see FIG. 3B) was 10 cm² and the volume of the recessed portion 12 was 25 cm³. Moreover, the flowrate of the carrier gas flowing through the inflow port 13 into the recessed portion 12 was set at 60 mL/min.

Firstly, it can be seen from the lower graph that the amount of ethanol in the skin gas from the palm P that was discharged together with the carrier gas rose in concert with the rise in the amount of ethanol in the exhaled breath. Here, points in time shown by thin, vertical straight lines in the graphs are points when the amount of sweat exceeded 0.1 mg/min after more than 10 minutes had elapsed since the alcohol was drunk. These points in time coincide with points when the amount of ethanol in the skin gas showed an abrupt peak. The reason for this is that, because the enzyme membrane 144 (see FIG. 2) that has been wetted by the buffer solution is not affected by moisture from sweat, it is possible to accurately measure not only the amount of ethanol contained in the skin permeable gas G1 shown in FIG. 6, but also the amount of ethanol contained in the sweat gas G2. In other words, if the amount of sweat increases, then the amount of ethanol contained in the sweat gas G2 also increases, and because this is added to the ethanol in the skin permeable gas G1, abrupt peaks are generated.

In this way, when a biosensor that utilizes an enzymatic reaction is used as the measurement device 100, it is possible to accurately measure the amount of ethanol in a living body using skin gas. In contrast, because it is possible to also measure the amount of ethanol in the sweat gas G2, abrupt peaks are evident in comparison with when the amount of ethanol in exhaled breath is measured, and, conversely, this may be problematic when estimating the amount of ethanol in blood. Therefore, in order to ascertain the amount of ethanol in blood from skin gas, the biological gas measurement device of the second exemplary embodiment measures the moisture within the recessed portion 12 using the moisture sensor 50, and is able to ascertain the amount of sweat by combining with the sweat meter 55. Moreover, the computer system 122 corrects the original data for the amount of ethanol measured by the measurement device 100 (for example, abrupt peaks in the amount of ethanol are smoothed out at points when the amount of sweat exceeds 0.1 mg/min) based on the measurements of the moisture (i.e., the amount of sweat). Because of this, effects of moisture (i.e., the amount of ethanol in the sweat gas G2 which is based on the amount of sweat) are excluded, and it becomes possible to more accurately measure the amount of ethanol in blood that has passed through skin gas.

In addition, it also becomes possible to use a semiconductor gas sensor whose characteristics may be changed by condensation and the like on the sensor surface, without using the measurement device 100 (i.e., the enzyme membrane 144 which is wetted with a solution) of the first exemplary embodiment. In other words, in the case of a semiconductor gas sensor, it is not possible to accurately measure the amount of ethanol in skin gas (in both the skin permeable gas G1 and the sweat gas G2), and there are also problems with the stability of the characteristics. However, in order to estimate the amount of ethanol in blood, it is sufficient if corrections are made based on the measurement results for the moisture (i.e., the amount of sweat) obtained by the moisture sensor 50 such that only original data at points in time when the characteristics were stable is utilized. Note that the method of correction may be one in which, for example, original data for the amount of ethanol at points in time when the amount of sweat exceeds a predetermined value is not used.

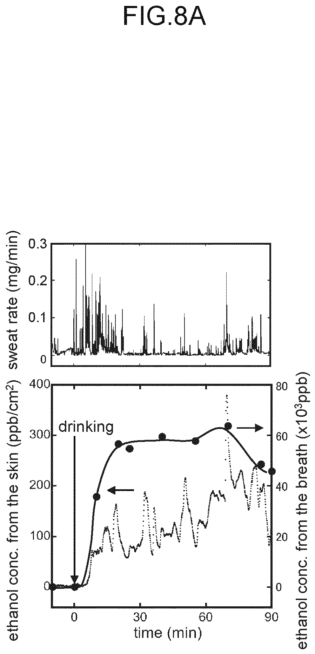

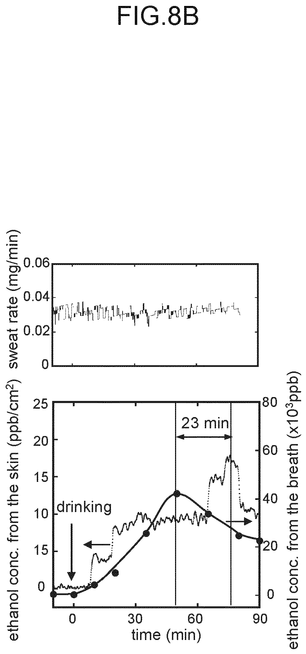

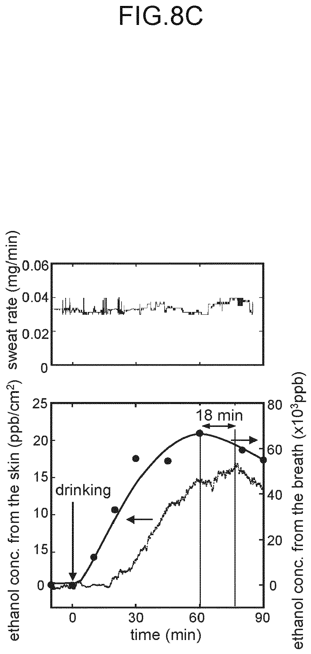

Furthermore, in FIG. 8A to FIG. 8C, the data (i.e., original data) obtained when the biological gas collector 10 was attached to the palm P (FIG 8A) shown in FIG. 7, for when the biological gas collector 10 was attached to the back of a hand (FIG 8B), and for when the biological gas collector 10 was attached to a wrist on the palm P side (FIG. 8C) are visualized as contrasting graphs. Firstly, when the biological gas collector 10 was attached to the back of a hand, compared with the palm P, the amount of sweat was less, and no large variations are seen. Moreover, it is evident that the amount of ethanol contained in skin gas from the back of the hand rises in concert with the rise in the amount of ethanol in exhaled breath, and that there is a delay of approximately 23 minutes until the amount of ethanol peaks. In contrast, when the biological gas collector 10 was attached to the wrist of a hand, compared with the palm P, the amount of sweat was still small, and no large variations are seen. However, it is evident that the amount of ethanol contained in skin gas and the amount of ethanol in exhaled breath have an extremely strong correlation, and that there is a delay of approximately 18 minutes until the amount of ethanol peaks. From the above, it can be understood that the measurement of the amount of ethanol on the palm P is affected by sweat, although not to any great extent, while the measurement of the amount of ethanol on the back of the hand and on the wrist (particularly, on the wrist) is substantially unaffected by sweat.

From these results, it may be said that it is preferable for the moisture sensor 50 to be provided when the amount of ethanol is measured on the palm P (particularly when a sensor other than a biosensor which utilizes an enzymatic reaction is used for the measurement device 100), and for a correction to be made by the computer system 122 based on the obtained measurements, so that effects of moisture (i.e., the amount of sweat) are excluded. Conversely, it may also be said that the moisture sensor 50 is not essential when the mount of ethanol is measured on the back of a hand or on the wrist (particularly when a biosensor which utilizes an enzymatic reaction is used for the measurement device 100), and that even though the amount of sweat (i.e., the sweat gas G2) is small, if it is still desired that effects thereof be excluded, then any small changes in the original data can be smoothed via processing performed by the computer system 122 so that measurements are corrected. Note that, in order to correct measurements of a target substance (more specifically, ethanol) performed by the measurement device 100 using measurements of sweat performed by the moisture sensor 50 using the biological gas measurement device according to the present exemplary embodiment, for example, excluding measurement points in time when the amount of sweat exceeded a predetermined amount, as described above, may be considered. In addition, a method in which values obtained by multiplying the amount of sweat by a predetermined coefficient are excluded from the measured ethanol amount values may also be considered.

### (3) Third Exemplary Embodiment

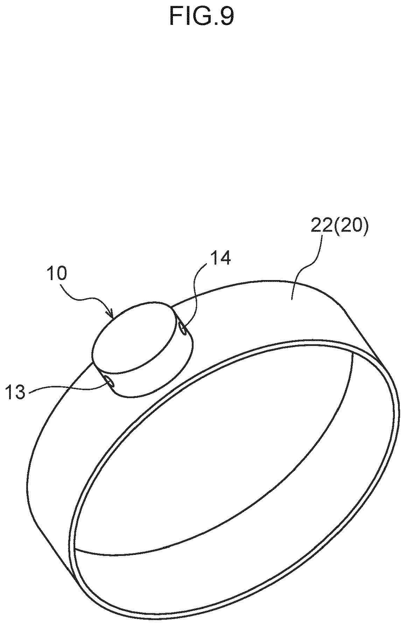

FIG. 9 is a view showing a third exemplary embodiment in which the biological gas collector 10 is fitted onto a wristband 22 which is serving as the adhering device 20. In the same way as in the above-described first and second exemplary embodiments, the inflow path 30 that is connected to the air supply device 130, and the outflow path 40 that is connected to the measurement device 100 are connected respectively to the inflow port 13 and the outflow port 14 of the biological gas collector 10. In addition, the recessed portion 12 is provided as a space for collecting skin gas. However, the recessed portion 12 of the third exemplary embodiment differs from that of the first and second exemplary embodiments in that, when attached to a wrist, the periphery of the aperture portion 11 is held tightly against the skin by a wristband 22 so that the recessed portion 12 is closed off from the outside air.

In the present exemplary embodiment, as shown in the previously mentioned FIG. 8C, the expandable wristband 22 is provided in order to provide an attachment to a wrist which is largely unaffected by sweat, and this enables the periphery of the aperture portion 11 of the biological gas collector 10 to be tightly adhered to the skin of the wrist. In other words, the third exemplary embodiment is characterized in being fitted onto a wrist. Because of this, it is not necessary to consider effects of sweat so that, unlike the second exemplary embodiment, the moisture sensor 50 is rendered unnecessary. Moreover, because there is essentially no moisture contained in the skin gas, it is also possible to measure the ethanol in skin gas using a semiconductor gas sensor, without using the measurement device 100 (i.e., the enzyme membrane 144 which has been wetted with a solution) of the first exemplary embodiment, and to only perform corrections so as to exclude effects of moisture in situations when this is necessary. Note that such situations might include when the wrist has become sweaty due to the person performing exercise or the like.

### (4) Fourth Exemplary Embodiment

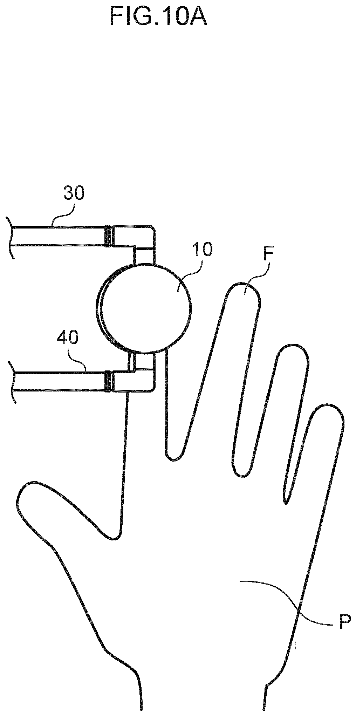

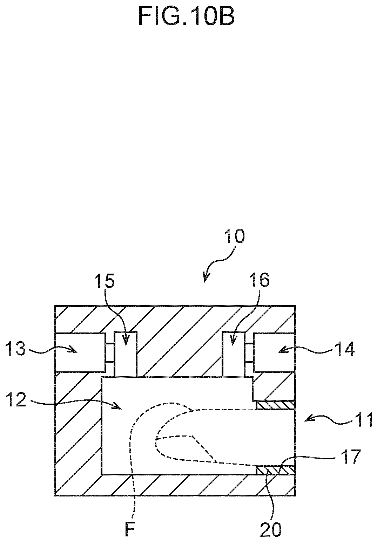

FIG. 10A is a view schematically showing a usage state of a fourth exemplary embodiment of the biological gas measurement device of the present invention, while FIG. 10B is a cross-sectional view of a biological gas collector. In the present exemplary embodiment, the amount of ethanol in skin gas is measured, not from a palm P of a hand, but from a pad portion of a fingertip by inserting a finger F into an aperture portion 11 (see FIG. 10B) which is provided in a side surface of the biological gas collector 10. Because of this, the adhering device 20 (see FIG. 10B) that is made from a closed cell foam body through which gas is unable to permeate is provided on a body contacting surface 17, which is an inner circumferential surface of the aperture portion 11, as a device for adhering to the finger F. When the finger F is inserted into the aperture portion 11, at least the pad portion of the fingertip faces the inflow communicating path 15 (i.e., a portion of the inflow path 30 described below) which communicates with the recessed portion 12, and the outflow connecting path 16 (i.e., a portion of the outflow path 40 described below) which also communicates with the recessed portion 12 (see FIG. 10B). Moreover, the inflow path 30 is connected to the inflow port 13 which communicates with the inflow communicating path 15, and the outflow path 40 is connected to the outflow port 14 which communicates with the outflow communicating path 16 (see FIG. 10A). Note that because the air supply device 130 that is connected to the inflow path 30, and the measurement device 100 that is connected to the outflow path 40 are the same as those of the first exemplary embodiment, a description thereof is omitted here.

### (5) Fifth Exemplary Embodiment

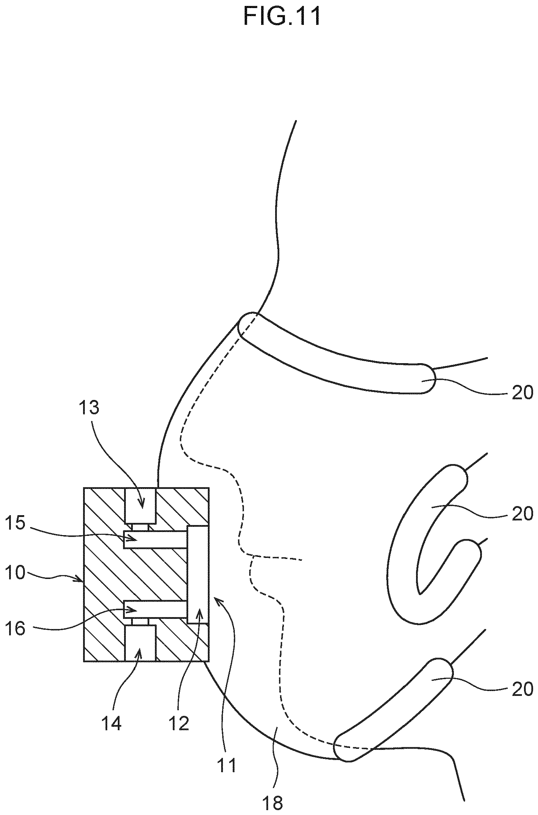

FIG. 11 is a view schematically showing a usage state of a fifth exemplary embodiment of the biological gas measurement device of the present invention. In the present exemplary embodiment, a mask 18 is fitted so as to cover the aperture portion 11 in the biological gas collector 10, and the amount of ethanol is measured using exhaled breath instead of skin gas as the biological gas. Because of this, the mask 18 is a device that covers the nose, cheeks, and chin, and is sufficiently flexible to provide a tight seal by following the contours of the face. The adhering device 20 that is made from a closed cell foam body through which gas is unable to permeate is provided, in particular, in locations on the peripheral edge of the mask 18 where there is a possibility of outside air coming in. In addition, when the mask 18 is being worn, the recessed portion 12 is located opposite the mouth of the person wearing the mask 18. Note that because the air supply device 130 that is connected to the inflow path 30 (not shown in the drawing) that is connected to the inflow port 13, and the measurement device 100 that is connected to the outflow path 40 (not shown in the drawing) that is connected to the outflow port 14 are the same as those of the first exemplary embodiment, a description thereof is omitted here.

### (6) Sixth Exemplary Embodiment

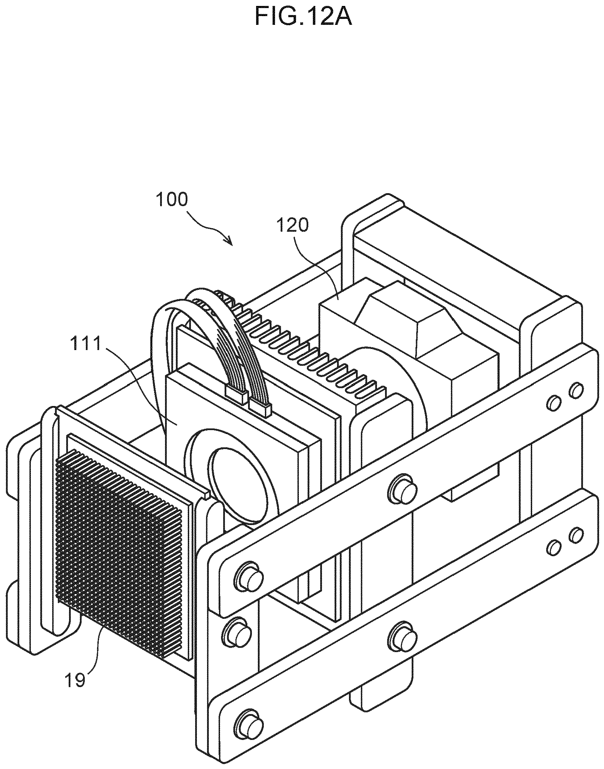

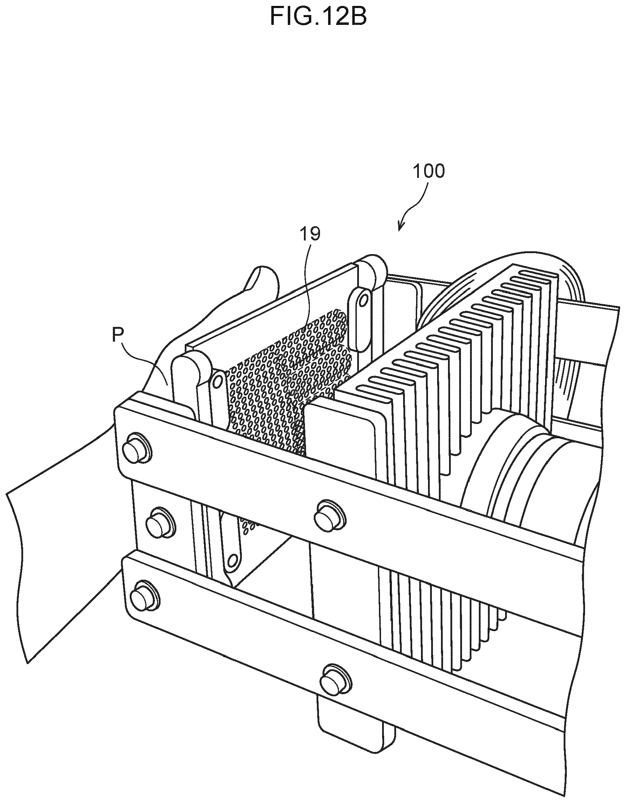

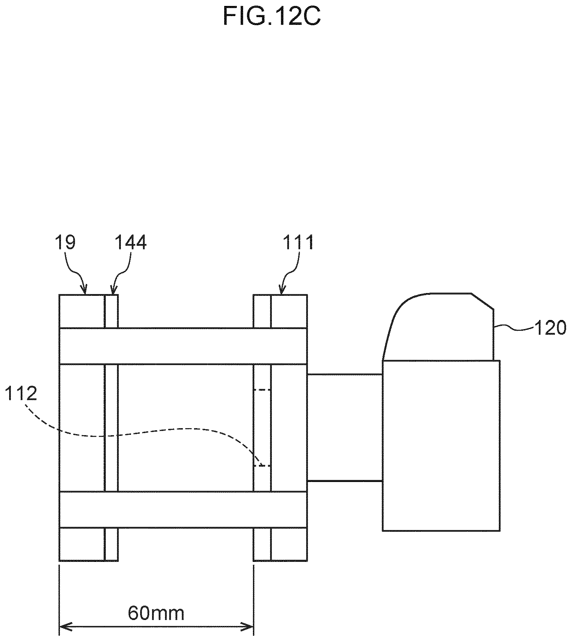

FIG. 12A to FIG. 12C are views respectively showing a sixth exemplary embodiment of the biological gas measurement device of the present invention in a front perspective view (FIG 12A), a back perspective view (FIG. 12B), and a side schematic view (FIG 12C). As shown in FIG 12A to FIG. 12C, in the sixth exemplary embodiment, a high-sensitivity camera ('ILCE-7S', manufactured by Sony Corporation), is used as the detection unit 120 in order to detect ethanol contained in skin gas, and a ring-shaped ultraviolet light-emitting diode is used as the excitation light irradiation unit 111. In addition, the light-receiving unit 112 (i.e., a high-sensitivity camera lens) and the detection unit 120 are provided on an inner side of the ring-shaped irradiation unit 111. Note that, in the biological gas measurement device of the present exemplary embodiment, the computer system 122 (see FIG. 1) that is connected to the detection unit 120 also functions as a visualization unit that analyzes detected data, and visualizes spatial distribution information of a concentration of the ethanol, which is the target substance of the present exemplary embodiment.

Moreover, there is also provided a two-dimensional surface profiler which serves as a clearance maintaining device 19 that maintains a clearance between the skin surface of the palm P of a hand and the enzyme membrane 144 at a fixed distance, and a PMMA plate is provided so as to maintain a clearance of 60 mm between the irradiation unit 111 and the front surface side of the clearance maintaining device 19. Note that, in FIG. 12B, the enzyme membrane which is provided at the back-surface side of the clearance maintaining device 19 (see FIG. 12C) is not shown.

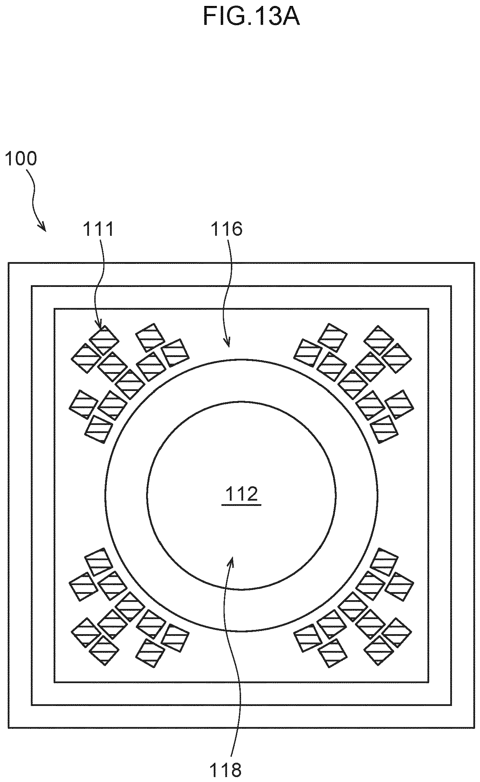

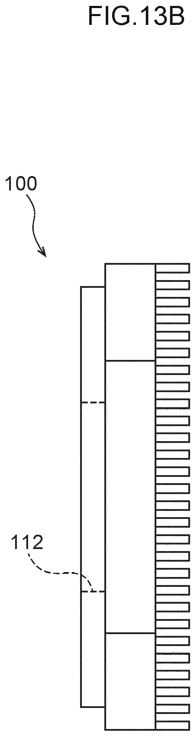

FIG. 13A and FIG. 13B are a front surface view (FIG. 13A) and a side surface view (FIG 13B) of the excitation light irradiation unit 111 and the light-receiving unit 112 for fluorescent light in the measurement device 100 which is used in the biological gas measurement device shown in FIG. 12A to FIG. 12C. As shown in FIG. 13A, the light irradiation unit 111 is formed so as to include a total of 40 ultraviolet light-emitting diodes (having a wavelength of 340 ± 5 nm sold by DOWA Electronics Materials Co. Ltd.) that are arranged in ring configurations in concentric circles having mutually different radiuses. A lens of a high-sensitivity camera (i.e., the detection unit 120) is then inserted into a through hole having a diameter of 75 mm in the center of the concentric circles so as to form the light-receiving unit 112. As a result, the irradiation unit 111, the light-receiving unit 112, and the detection unit 120 are integrated into a single unit. Moreover, a bandpass filter for excitation light (having a wavelength of 340 ± 42.5 nm manufactured by HOYA Corporation) is then disposed as the bandpass filter 116 in the front surface of the irradiation unit 111, and a bandpass filter for fluorescent light (having a wavelength of 492 ± 10 nm manufactured by Edmond Optics Japan) is then disposed as the long wavelength transmission filter 118 in the front surface of the light-receiving unit 112 so as to be on the same plane therewith. In this way, by affixing the ring-shaped irradiation unit 111 to the outer periphery of the light-receiving unit 112 (i.e., by providing light-receiving unit 112 on the inner side of the irradiation unit 111), the irradiation unit 111 and the light-receiving unit 112 can be positioned on a same side as the enzyme membrane 144, and the irradiation of excitation light in the direction of the enzyme membrane 144 can be performed in the same direction as the receiving of the fluorescent light. Accordingly, because the excitation light irradiated towards the enzyme membrane 144 during the measurement of the fluorescent light is not obstructed by the body, a visual form of the ethanol contained in skin gas may be provided continuously and in real time by the visualization unit.

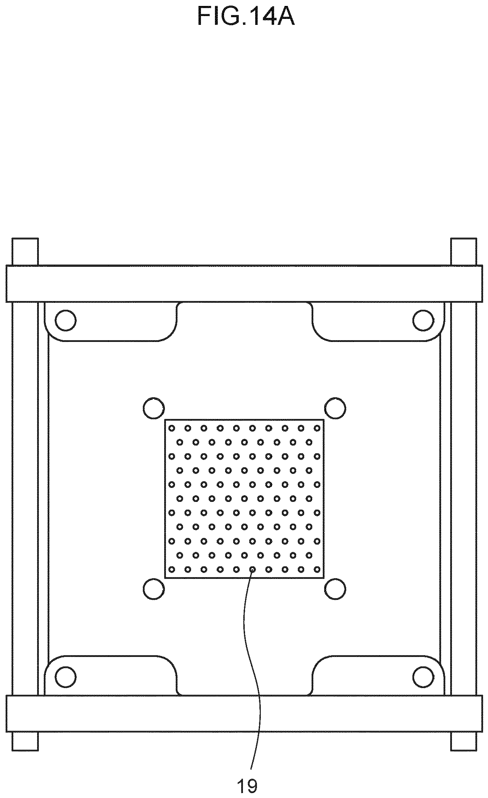

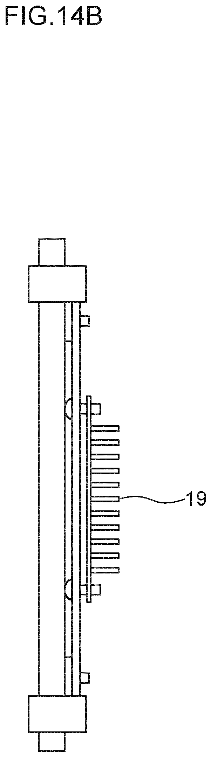

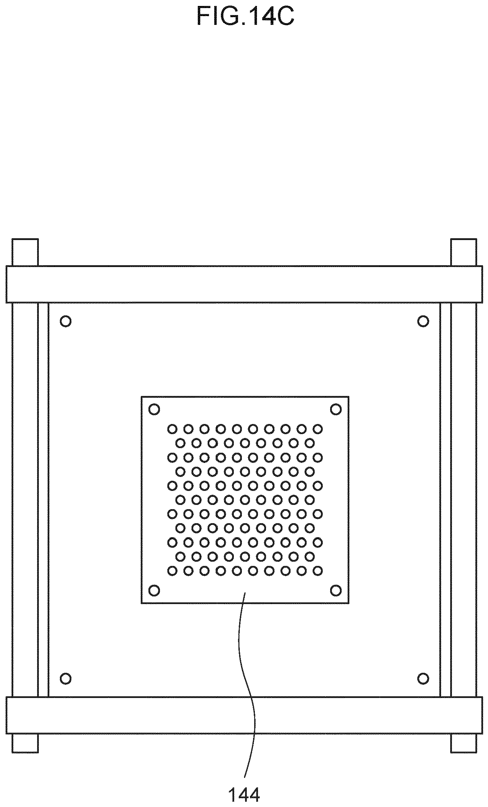

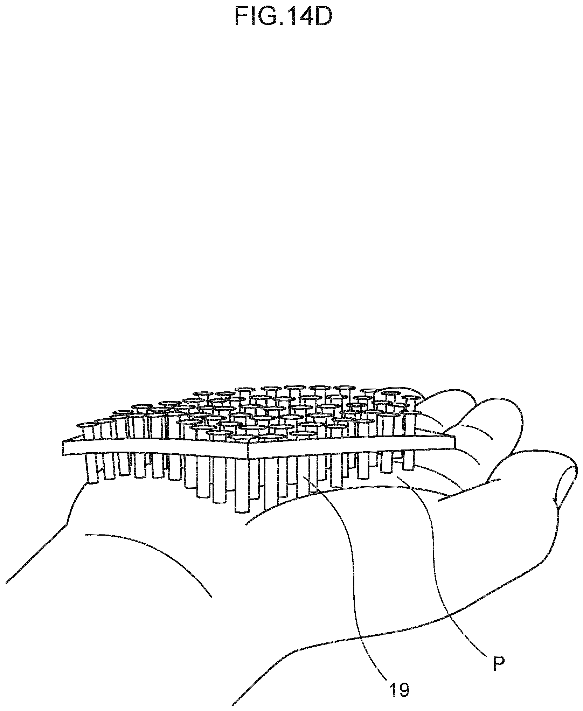

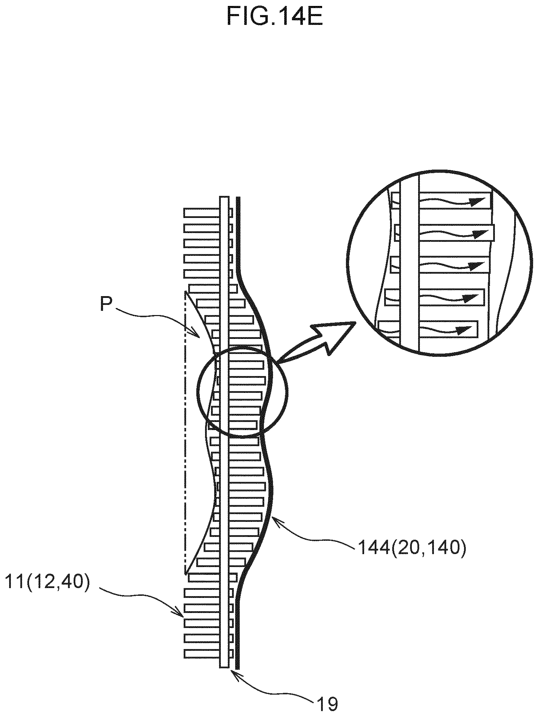

In order to provide a high-accuracy visualization of the ethanol in skin gas, it is necessary for the clearance between the skin and the enzyme membrane 144 to be kept constant over the contours of the palm P. FIG. 14A to FIG. 14E show the clearance maintaining device 19 that is used in the biological gas measurement device shown in FIG. 12A to FIG. 12C in a front view (FIG 14A), a side view (FIG 14B), a back view (FIG 14C), a perspective view showing a usage example (FIG 14D), and in a side view during use (FIG 14E). The two-dimensional surface profiler serving as the clearance maintaining device 19 is formed by 594 stainless pipes (having an outer diameter of 2.5 mm, an inner diameter of 2.3 mm, and a length of 10 mm) that penetrate the same number of through holes (having a diameter of 2.6 mm) in a PPMA plate (120 mm x 120 mm in area and having a thickness of 3 mm). A distal end of each pipe, which is on the palm P side, corresponds to the aperture portion 11, while the pipe interiors correspond to the recessed portions 12, and the overall structure forms an exemplary embodiment of a biological gas collector. Furthermore, a rear end of each pipe forms an embodiment of the outflow path 40 through which skin gas is discharged to the enzyme membrane 144 forming a portion of the measurement device 100.

The enzyme membrane 144 is formed by using 100% cotton mesh (having a size of 90 x 90 mm to match the size of the palm P, a thickness of 1 mm, and a pitch of 1 mm) having no autofluorescence as a support body, and immobilized ADH as the enzyme. The enzyme membrane 144 is attached to the rear end of each pipe of the clearance maintaining device 19. Because of this, when the palm P is pressed against the clearance maintaining device 19, as shown in FIG. 14D and FIG. 14E, the aperture portion 11 at the distal end of each pipe is in contact with the palm P so that the enzyme membrane 144 is deformed, and functions as the adhering device 20 due to the elastic force thereof. Moreover, the clearance between the aperture portions 11 and the enzyme membrane 144 is kept constant.

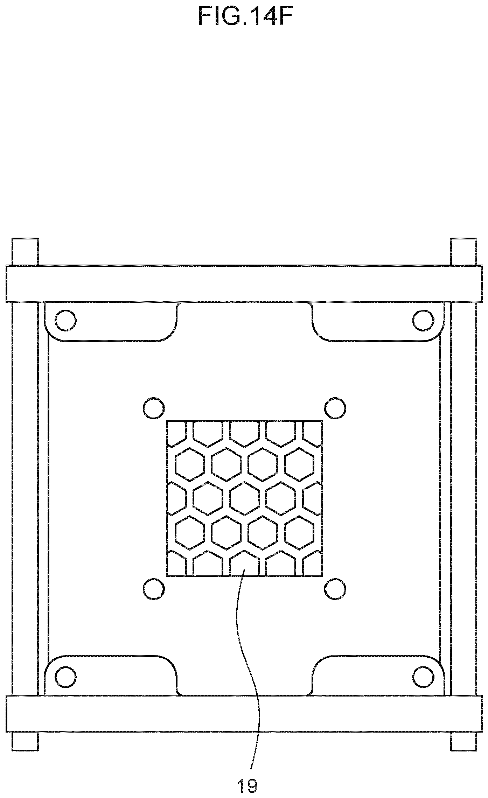

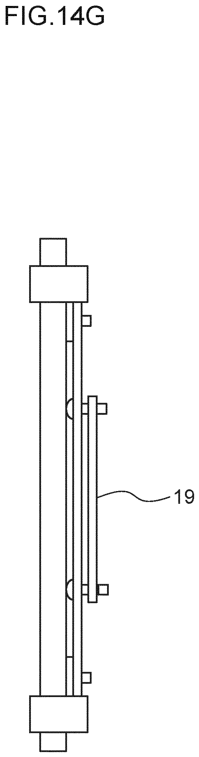

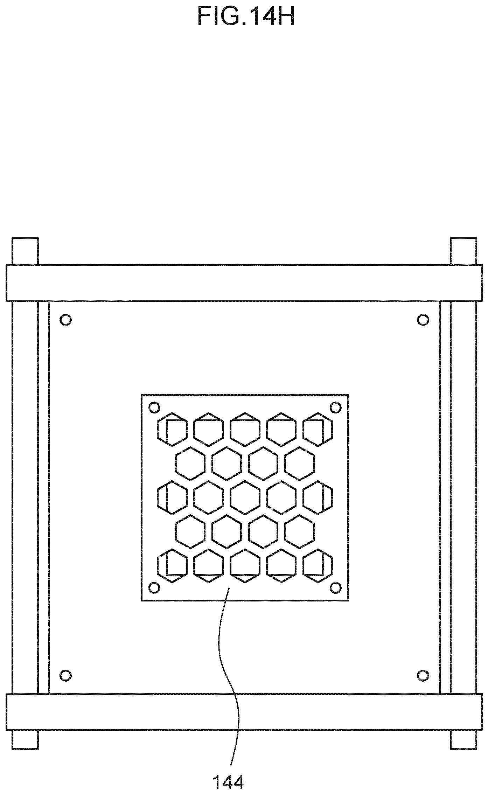

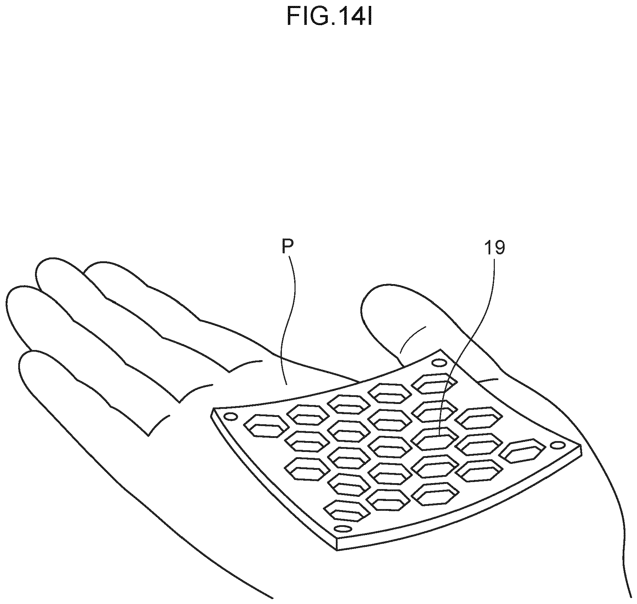

Furthermore, because the support body (i.e., the cotton mesh) of the enzyme membrane 144 is filled with a solution containing NAD⁺, this support body functions as the container body 140. Because of this, fluorescent light is generated by the excitation light irradiated from the irradiation unit 111 in the direction of the enzyme membrane 144, and this fluorescent light is received by the light-receiving unit 112. Note that the clearance maintaining device 19 may also take a form such as that shown in a front view (FIG 14F), a side view (FIG 14G), and a back view (FIG 14H), and in a perspective view showing a usage example (FIG 14I), which each show another example thereof. More specifically, a plurality of holes are formed in a flexible planar body made from polydimethylsiloxane (PDMS), with each hole performing the same role as the respective pipes of the two-dimensional surface profiler. As shown in FIG. 14I, this clearance maintaining device 19 is able to change so as to conform to the contours of the palm P. Moreover, because the distance from the palm P to the enzyme membrane 144 is close (i.e., because each pipe is short or because the plate body is thin), the air supply device 130 (see FIG. 1) is rendered unnecessary.

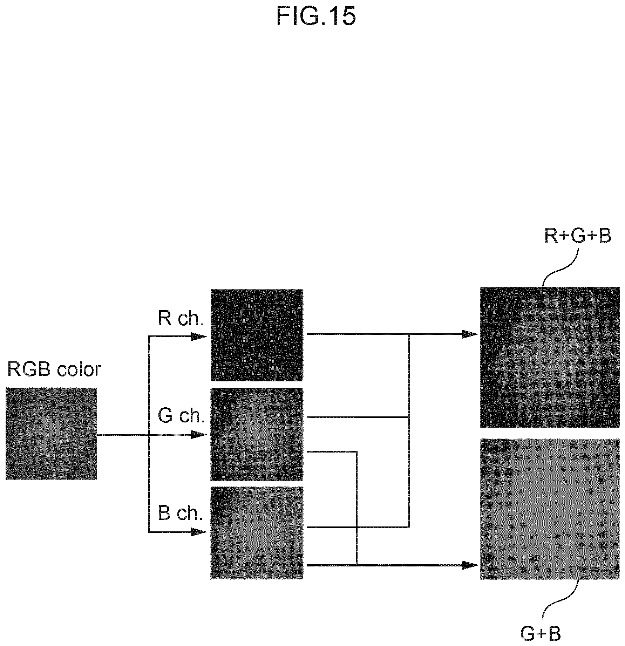

FIG. 15 is a view schematically showing a method of providing a high-sensitive visual form of an amount of ethanol contained in skin gas using the biological gas measurement device shown in FIG. 12A to FIG. 12C. As shown in FIG. 15, by using an RGB camera which is capable of acquiring an RGB color image as the detection unit 120 shown in FIG. 12A to FIG. 12C, it is possible to detect the fluorescent intensity released from the NADH at a higher S/N ratio by performing image analysis. More specifically, of the RGB color image received by the lens (i.e., the light-receiving unit) of the RGB camera, a bicolor image (i.e., the 'G + B' in the drawings) is obtained by adding only a G channel and a B channel that have sensitivity at 491 nm, which is the fluorescence wavelength of NADH. Using this bicolor image, the aforementioned type of detection is possible. As a result, compared with the normal method (i.e., the 'R + G + B' in the drawings. 0.299 × R + 0.587 × G +0.114 × B) in which an optional coefficient is multiplied by the R channel, the G channel, and the B channel using the high-sensitivity camera used in FIG. 12A to FIG. 12C, and then adding these together, it becomes possible to detect ethanol gas having a concentration of approximately 1/50. In addition, a determination range of 0.01 to 300 ppm, and a wide dynamic range can also be obtained.

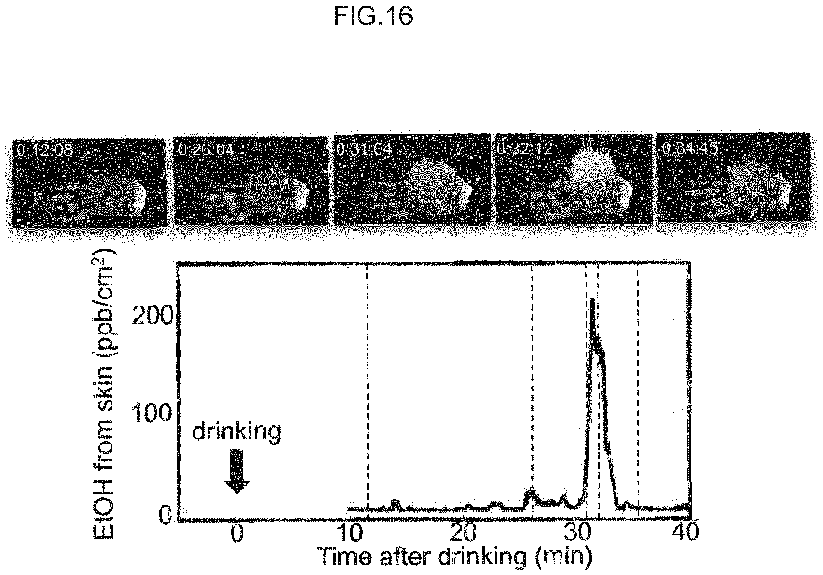

FIG. 16 is a view schematically showing an amount of ethanol contained in skin gas that has been provided in a visualized form using the biological gas measurement device shown in FIG. 12A to FIG. 12C. Firstly, alcohol drinks (25% proof) were orally ingested (for 15 minutes) such that the alcohol drinking conditions were 0.4g of ethanol per kilogram of body weight. Next, for the 30 minutes between 10 and 40 minutes after the alcohol was drunk, the two-dimensional surface profiler which was serving as the clearance maintaining device 19 was pressed against the palm P of the hand of the test subject. The quantity of ethanol contained in the released skin gas was continuously visualized and measured. As a result, chronological changes in the concentration of ethanol released from the skin after drinking were obtained in a visualized form. As shown in FIG. 16, the chronological changes which reached a peak value 32 minutes after drinking were obtained as real-time video images. Moreover, the obtained video images were rendered to a differential analysis, and the chronological changes in concentration per 1 cm² of surface area of the palm P were calculated based on a calibration curve. Note that the spike-shaped concentration changes visible in FIG. 16 are considered to be associated with the sweat (i.e., ethanol contained in the sweat gas G2 (see FIG. 6)) generated from the palm P.

### (7) Seventh Exemplary Embodiment

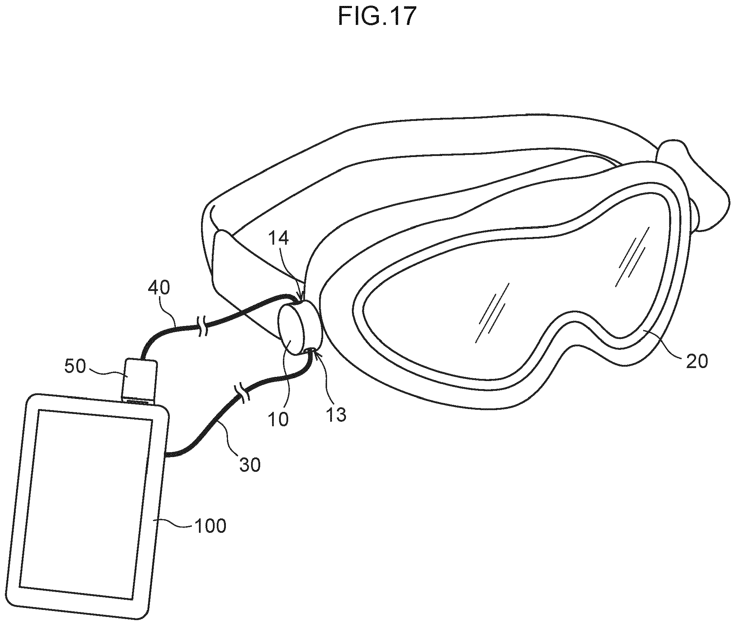

FIG. 17 is a view schematically showing a seventh exemplary embodiment of the biological gas measurement device of the present invention. In the present exemplary embodiment, the biological gas collector 10 is fitted onto one end of goggles which are serving as the adhering device 20 that covers an area around the eyes. The amount of ethanol contained in gas released from the eyeball and conjunctiva, as well as skin gas released from the eyelids, and also evaporation gas from tears is measured by this biological gas collector 10 as biological gas. Because of this, the adhering device 20 covers both eyes and is sufficiently flexible to follow the contours of the face, and is also formed so as to provide a tight seal at locations on the peripheral edge of the adhering device 20 where there is a possibility of outside air coming in. Note that the inflow path 30 that is joined to the inflow port 13 of the biological gas collector 10 is connected to the air supply device 130 (see FIG. 1) that is incorporated into the measurement device 100, and the outflow path 40 that is connected to the outflow port 14 is connected in the same way to the measurement device 100 via the moisture sensor 50. The measurements performed by the measurement device 100 are performed in the same way as in the first exemplary embodiment.

### (8) Eighth Exemplary Embodiment

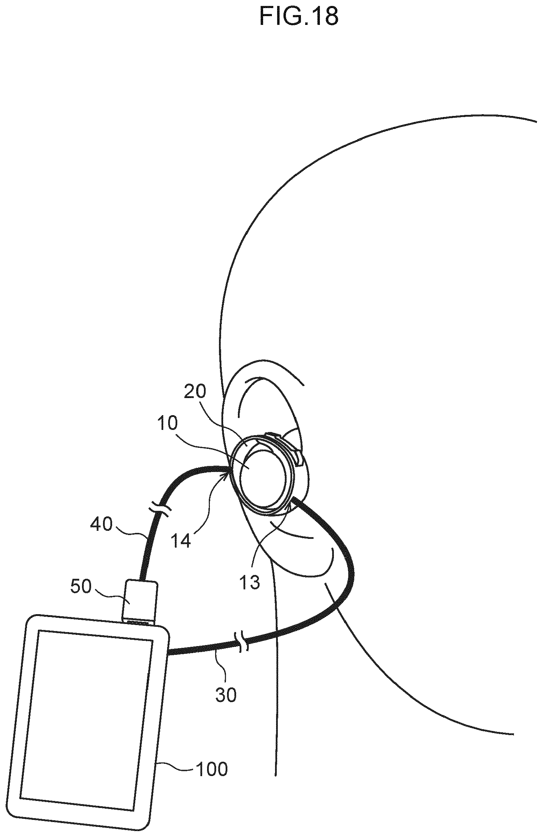

FIG. 18 is a view schematically showing an eighth exemplary embodiment of the biological gas measurement device of the present invention. In the present exemplary embodiment, the biological gas collector 10 is fitted inside the adhering device 20 which is shaped like an earphone that is fitted inside an ear canal. The amount of ethanol contained in skin gas released from the ear canal as biological gas is measured by this biological gas collector 10. Because of this, the adhering device 20 covers the ear canal and is sufficiently flexible to follow the contours of the pinna, and is also formed so as to provide a tight seal at locations on the peripheral edge of the adhering device 20 where there is a possibility of outside air coming in. Note that the inflow path 30 that is joined to the inflow port 13 of the biological gas collector 10 is connected to the air supply device 130 (see FIG 1) that is incorporated into the measurement device 100, and the outflow path 40 that is connected to the outflow port 14 is connected in the same way to the measurement device 100 via the moisture sensor 50. The measurements performed by the measurement device 100 are performed in the same way as in the first exemplary embodiment.

Although exemplary embodiment of the biological gas measurement device of the present invention have been described above, the present invention is not limited to these exemplary embodiments and various modifications and the like may be made thereto. For example, in addition to a palm or finger of a hand, a wrist, and a face and the like, the above-described biological gas collector may also be attached to the skin of various body portions such as a leg, an arm, a neck, or a waist or the like, or to lips. In addition, the above-described biological gas collector may also be integrated with a wristband, a wristwatch, a mask, goggles, earphones, headphones, or hearing aids or the like so as to form a wearable terminal, or else may be incorporated into bodyweight scales or a blood pressure meter or the like. Furthermore, in addition to ethanol, the above-described biological gas collector may also be used to measure acetone and the like.

In the case of acetone, the acetone is discharged to the outside of the body via blood as skin gas or exhaled breath, and by then measuring the concentration thereof, it is possible to evaluate a fat combustion condition, or how far diabetes has progressed or the like. For example, if the biological gas measurement device of the present invention that is capable of outputting measurement results for acetone from which effects of moisture have been excluded is incorporated into body weight scales, then acetone can be measured through the soles of the feet as part of an everyday action such as getting on the scales after taking a shower or the like. In this case, it is possible to accurately determine whether any decrease in weight is due to a decrease in body fat, and to thereby determine whether a diet is effective or not. Accordingly, this can lead to the elimination and prevention of obesity which is the basis of many lifestyle-related diseases. Moreover, the biological gas measurement device of the present invention may also be fitted onto an arm or the like of a person who is exercising on a running machine and generating sweat, and used to measure the acetone in such a situation so that fat metabolism is visualized. Furthermore, the biological gas measurement device of the present invention may also be used to measure *trans*-2-nonenal which is one of the substances that cause odors in elderly people, so that it also becomes possible to evaluate changes in metabolic function that accompany the aging process.

## Claims

1. A biological gas measurement device comprising:
a biological gas collector having an aperture portion in a side thereof that faces a living body, and having a recessed portion that is connected with the aperture portion and serves as a space for collecting biological gas released either directly or indirectly from the living body;
a measurement device that measures a target substance in the biological gas collected by the biological gas collector;
an outflow path through which collected biological gas is discharged from the recessed portion to the measurement device;
a moisture sensor that measures moisture present inside the recessed portion or the outflow path; and
a correction device that corrects measurements of the target substance performed by the measurement device, using measurements of moisture performed by the moisture sensor, and that enables measurement results of the target substance, from which effects of moisture present inside the outflow path have been excluded to be output.

2. The biological gas measurement device according to claim 1, further comprising:
an inflow port through which a gas flows into the recessed portion from outside;
an outflow port through which a gas is discharged from the recessed portion to the outside; and
an air supply device that supplies a carrier gas from the inflow port to the recessed portion via an inflow path,
wherein the outflow path guides the biological gas discharged together with the carrier gas from the outflow port to the measurement device.

3. A biological gas measurement device comprising:
a biological gas collector having an aperture portion in a side thereof that faces a living body, and having a recessed portion that is connected with the aperture portion and serves as a space for collecting biological gas released either directly or indirectly from the living body;
a measurement device that measures a target substance in the biological gas collected by the biological gas collector;
an outflow path through which collected biological gas is discharged from the recessed portion to the measurement device; and
a correction device that corrects measurements of the target substance performed by the measurement device, and that enables measurement results of the target substance, from which effects of moisture present inside the outflow path have been excluded, to be output, wherein:
the measurement device comprises:
an enzyme membrane that is fitted onto an adjacent portion relative to the outflow path, at least a portion of the enzyme membrane being wetted by a solution containing a coenzyme, and the wetted portion being exposed to the biological gas and the moisture present inside the outflow path; and
an irradiation unit that irradiates excitation light of a predetermined wavelength onto the solution,
an enzyme that catalyzes a chemical reaction of the target substance which accompanies a chemical change in the coenzyme is fixed to the enzyme membrane,
the coenzyme emits fluorescent light when excited by the excitation light, and
the target substance is measured by fluorescence detection before and after the chemical change in the coenzyme, so that effects of moisture can be excluded by the correction device.

4. The biological gas measurement device according to claim 3, further comprising a clearance maintaining device that maintains a clearance between the aperture portion and the enzyme membrane at a fixed distance.

5. The biological gas measurement device according to claim 3 or claim 4, wherein the measurement device further comprises a light-receiving unit that receives the fluorescent light.

6. The biological gas measurement device according to claim 5, wherein:
the irradiation unit is ring-shaped, and
the light-receiving unit is provided on an inner side of the irradiation unit, and the irradiation unit and the light-receiving unit are positioned on a same side relative to the enzyme membrane.

7. The biological gas measurement device according to claim 5 or claim 6, further comprising a detection unit that detects the target substance using fluorescent light received by the light-receiving unit.

8. The biological gas measurement device according to claim 7, wherein the detection unit detects the target substance by adding only a G channel and a B channel of an RGB color image received by the light-receiving unit.

9. The biological gas measurement device according to claim 7 or claim 8, further comprising a visualization unit that visualizes spatial distribution information of a concentration of the target substance detected by the detection unit.

10. The biological gas measurement device according to any one of claim 1 to claim 9, further comprising an adhering device that causes a periphery of the recessed portion to adhere to the living body.
